# EUROPEAN PATENT SPECIFICATION

(11) **EP 2 306 894 B1**
(45) Date of publication and mention of the grant of the patent: **13.06.2012**
(21) Application number: 09772802.6
(22) Date of filing: 01.07.2009
(51) Int. Cl.: A61B 5/145, A61B 5/1477, A61N 1/30

(54) **PATCHES FOR REVERSE IONTOPHORESIS**
PFLASTER FÜR REVERSE IONTOPHORESE
TIMBRES POUR IONTOPHORESE INVERSE

(30) Priority: 30.06.2008 GB 0811874; 26.01.2009 GB 0901188
(43) Date of publication of application: 13.04.2011
(73) Proprietor: Nemaura Pharma Limited, Leicestershire LE11 1PN (GB)
(72) Inventor: CHOWDHURY, Dewan, Fazlul, Hoque, Leicestershire LE11 1PN (GB)
(74) Representative: Smith, Peter James
(86) International application number: PCT/GB2009/001652
(87) International publication number: WO 2010/001122

(56) References cited:
- WO-A1-2005/120471
- US-A- 5 310 404
- US-A- 5 827 183
- US-A1- 2002 002 328
- US-B1- 6 391 643

## Description

### Technical field

The invention relates to patches for applying to the skin of a patient, whereby constituents of fluids in the skin for analysis can be withdrawn through the skin by the technique of reverse iontophoresis. Furthermore it relates to mechanisms for enhancing the sensitivity, reliability and accuracy of analysis of the said analytes.

The term "drug" is used in this specification to refer to any biologically active substance that needs to be delivered into the bloodstream of the patient, whether therapeutic or not, for example pharmaceuticals, vaccines and proteins. The patient may be human or animal.

### Background of the invention

The technique of iontophoresis is known for delivery of drugs through the skin of a patient. A pair of electrodes is applied to the skin and one of them is used to repel charged molecules of a drug in order to drive them into the body of the patient through pores in the skin. It is also known to operate the process in reverse, in order to draw ions, molecules or other components of the interstitial fluid out of skin for analysis. Interstitial fluid is fluid found between cells in the extracellular spaces, the main constituents of which are water, amino acids, sugars, fatty acids, co-enzymes, hormones, neurotransmitters, drugs (administered to a patient), salts and waste products from cells. Interstitial fluid differs from whole blood in that red blood cells are absent, and there are far fewer proteins present.

United States patent application US 2002/0002328 A1, which is assigned to Cygnus Inc., discloses one example of a method and device for sampling substances through the skin of a patient by reverse iontophoresis. The electrode is suspended in the centre of a sampling chamber to be surrounded by liquid during use of the device and the electrode may alternate its function between serving as the anode and the cathode. Devices such as the Glucowatch^{®} device are known that withdraw analytes from interstitial fluid at periodic intervals for the purpose of checking the glucose level of a diabetes sufferer. (Glucowatch is a registered trade mark of Cygnus, Inc.) The Glucowatch^{®} device uses the process of reverse iontophoresis to draw analytes such as glucose from the skin. The analyte is drawn on to a gel pad from where it is reacted with glucose oxidase to form hydrogen peroxide, the concentration of which is determined electrochemically. As described in U.S. patent 6,391,643, which is assigned to Cygnus Inc., the gel pad may be separated from the iontophoresis electrodes by a liner sheet prior to application of the device to the skin of a patient. The concentration of glucose drawn from the skin is in the region of one thousandth of that present in interstitial fluids, thus requiring a very sensitive assay/detection method and a sensor extending over substantially the whole area of the gel pad. Moisture or sweat on the skin causes the device to malfunction and a warm up period of 2 hours is required after application of the device to the skin, prior to measurement of the glucose levels. The Glucowatch^{®} device is typically strapped to the wrist of a patient in the manner of a wristwatch and loss of contact of the gel pad with the skin, and loss of conductivity and iontophoretic mobility is common, in particular due to natural movements of the body and skin contour. Furthermore electromigration (or the movement of an analyte driven by electromotive force) has greater resistance in a higher viscosity medium such as a gel as compared to a liquid medium, thus leading to the need for greater potential differences or increased duration of application of electrical currents to drive the molecules to the sensor from within the skin. There are also limitations to the sensor technique that may be employed where the analyte is collected on to a gel pad, and there will be issues with respect to further concentrating the analyte for detection in any specific region, requiring routine calibration of the sensor. In this case the autosensor has to be discarded and replaced with a new one each time the device is removed from the skin (i.e., each time contact with the skin is broken). The device is also affected by very cold weather and has the propensity to cause skin irritation (and it has been reported to have caused skin irritation in up to 25% of all users).

### Summary of the invention

The invention as defined in claim 1 provides a patch for sampling one or more analytes through the skin of a patient comprising:
an electrode layer for positioning adjacent to the skin of a patient;
means for actuating the electrode layer to induce the withdrawal of analyte through the skin of the patient by reverse iontophoresis;
a first reservoir containing an electrically conducting medium such as a liquid electrolyte; and
means for controlling transport of the conducting medium from the first reservoir onto a surface of the electrode layer adjacent to the skin to increase the conductivity between the electrode layer and the skin; and
means for transporting analytes withdrawn from the skin of the patient to a location where they are to be analysed.

The conducting medium is preferably a liquid electrolyte. The means for controlling transport of the conducting medium from the reservoir may comprise either means for applying negative pressure to a channel downstream from the reservoir or means for applying positive pressure to the reservoir itself. The means for applying positive pressure may comprise an actuator means, which actuates on receipt of a control stimulus to stretch and/or compress the reservoir. If the patch is adhered to the skin of the patient, the actuation of the actuator means may also cause extension of the skin for enhanced transport of the analyte through the skin.

The means for transporting analytes to a location where they are to be analysed may merely increase the concentration of analytes in a region of the sampling chamber adjacent to the skin but it preferably comprises a conduit for delivering the analytes to a separate sensor chamber. This preferably does not entail bulk movement of the fluid away from the sampling chamber but rather the selective transport of the analyte molecules or ions of interest through the fluid. Concentration of the analytes in a restricted location greatly enhances the capability of the sensors to detect them.

A patch according to the invention may further comprise a second reservoir containing a drug for transdermal delivery to the patient, wherein the means for controllably delivering electrolytic liquid from the first reservoir is also suitable for delivering the drug from the second reservoir to the skin of the patient. The delivery of the drug may be controlled automatically based on the results of the analysis of the analytes withdrawn from the patient by the patch.

The invention further provides a method as defined in claim 13 of sampling one or more analytes through the skin of a patient comprising the steps of:
positioning a patch such that an electrode layer of the patch is adjacent to the skin of the patient;
transporting an electrically conducting medium from a first reservoir in the patch onto a surface of the electrode layer adjacent to the skin to increase the conductivity between the electrode layer and the skin;
actuating the electrode layer to induce the withdrawal of analyte through the skin of the patient y reverse iontophoresis; and
transporting the analytes withdrawn from the skin of the patient to a location where they are to be analysed.

### The drawings

Figures 1a and 1b are schematic cross sections through a reverse iontophoresis patch according to the invention, respectively in a relaxed state and in an active state.
Figures 2a and 2b are schematic cross sections through a single reservoir of a reverse iontophoresis patch according to the invention, respectively in a relaxed state and in an active state.
Figures 2c and 2d are schematic cross sections similar to Figures 2a and 2b but showing details of alternative means for analyte collection.
Figure 3 is a drawing of an actuator formed from a shape memory alloy sheet that is suitable for use in a patch in accordance with the invention.
Figure 4 is a drawing of an alternative actuator formed from shape memory alloy wire that is suitable for use in a patch in accordance with the invention.

### Description of preferred embodiments

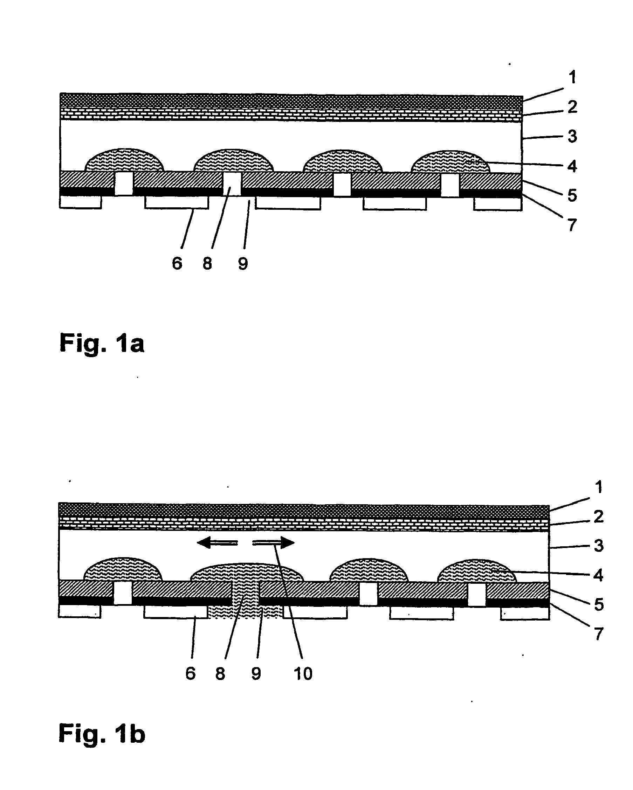

Figures 1a and 1b show schematically a cross section of a small part of a patch for reverse iontophoresis. The relative thickness of the respective layers is not to scale. In practice the area of the patch may be a few square centimetres and its thickness no more than a few millimetres so that it is flexible enough to flex with the skin of a patient.

The patch comprises a reservoir layer 3 that consists of a series of chambers 4 containing one or more liquids for delivery to the skin of a patient. As discussed below, the liquids may include drugs or electrolyte solutions. The reservoir layer 3 is flexible and its lower surface is bounded by a resilient membrane 5, which is perforated by pores 8 through which liquids can pass from the reservoir chambers 4 to the skin. An adhesive layer 6 removably bonds the patch to the skin of the patient. The adhesive layer 6 may be generally permeable to liquids or may include pores aligned with the pores 8 in the resilient layer 5, as shown.

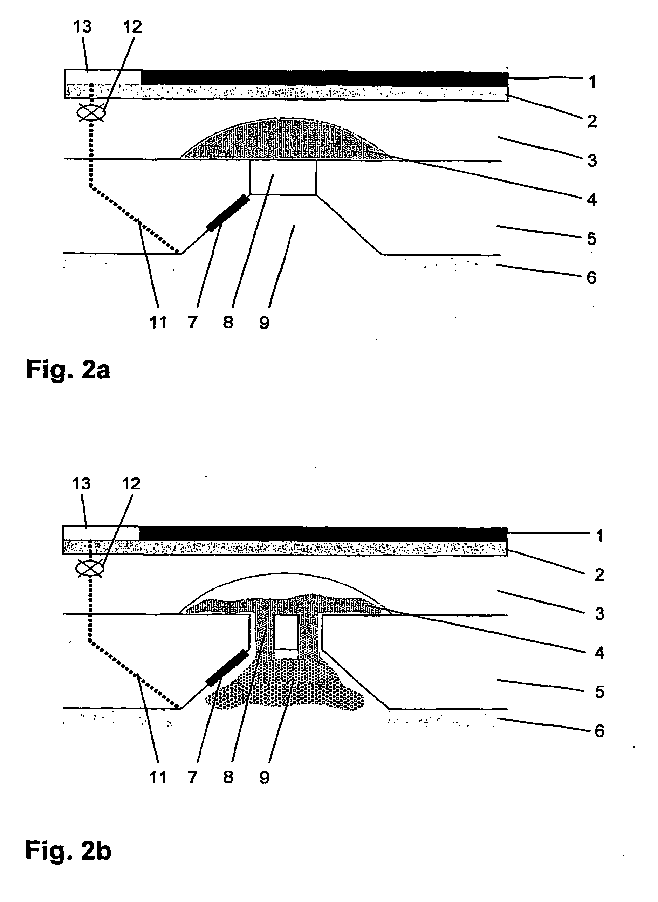

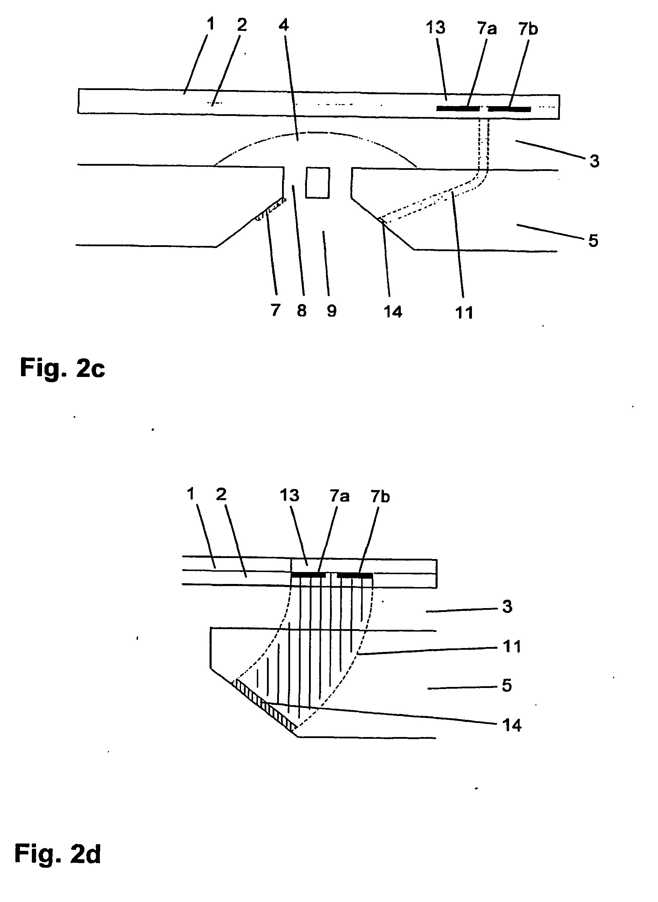

An upper surface of the reservoir layer 3 is attached to an actuator layer 2, which is typically formed as a microelectromechanical (MEMS) device. The actuator layer 2 is in turn attached to an upper control layer 1 comprising microelectronic control circuitry for the patch. The patch includes analysis chambers 13 for fluids withdrawn from the patient (shown in Figure 2 but not Figure 1), which may also be located in the control layer 1, along with associated sensors and processors. Conduits 11 for the analytes pass through the thickness of the patch from the skin-facing surface to the analysis chambers 13.

An electrode layer 7 is formed on a surface of the patch adjacent to the skin of the patient. The electrodes 7 may be formed as thin films of silver, silver chloride, carbon nanotubes or other suitable materials , for example by printing onto the polymer film of the resilient layer 5 using ink-jet technology. The electrodes are patterned to form an array of anodes and cathodes suitable for reverse iontophoresis. By the application of appropriate voltages and currents to the skin through the electrodes, components of the interstitial fluids may be drawn out of the skin of the patient and through the conduits 11 into the chambers 13 for analysis. The patch is thus suited to continuous or intermittent monitoring of the levels of indicative substances in the interstitial fluids/skin of the patient.

Operation of the actuator 2 of the patch under the control of the microelectronics layer 1 causes it to alternately extend and relax. In turn, this alternately extends and relaxes the reservoir layer 3, the resilient membrane 5 and the area of the patient's skin to which the patch is adhered. Stretching of the surface layer of the skin - the *stratum corneum -* results in disruption of the skin surface barrier, and stretching of pores such as sweat pores and hair follicles results in enhancement of pore diameters, thus enhancing the transport of interstitial fluids through the skin into the patch.

In accordance with the invention, at least some of the reservoir chambers 4 contain an electrically conducting medium (hereinafter referred to as an 'electrolyte'), and may include a buffered salt solution, as described in literature, which is required to induce the process of iontophoresis. Operation of the actuator to extend the reservoir layer 3 has the effect of deforming the reservoir chambers 4 - typically by stretching them in one dimension and compressing them in the others - so that their volume is reduced and the electrolyte is forced out of the chambers 4 and through the pores 8 in the resilient membrane 5 towards the skin of the patient. As shown particularly in Figure 2, the resilient membrane 5 may be shaped to form a cavity 9 between the electrodes 7 and the skin of the patient, into which the electrolyte can flow. The presence of the electrolyte in liquid form ensures good conductivity between the electrodes and the skin, which greatly enhances the effectiveness of the reverse iontophoresis process.

All of the above listed problems with existing systems such as the Glucowatch^{®} device are overcome through this invention. Enhanced safety is achieved through reduced skin irritation, and no loss of electrical contact due to abrupt body movements, thus making the system more reliable due to the liquid (low viscosity) nature of the conducting medium. In the event of loss of contact with the skin the excess fluid may be removed from the skin and the device re-applied and the operation can continue by virtue of a new reservoir being actuated to release the conducting medium to make electrical contact with the skin (based on an appropriate microelectronic control program). Skin irritation may be reduced further using materials in the conducting medium that are known to inhibit skin irritation in human and animals. These materials include alkali metal bases, aloe vera, chamomile, alpha-bisabolol, Cola nitida extract, green tea extract, tea tree oil, liquorice extract, allantoin, urea, caffeine or other xanthines, glycyrrhizic acid and its derivatives and the divalent cation strontium, antihistamines and other anti-pruritic agents amongst others.

The amount of analyte drawn from the skin may also be further enhanced by the incorporation of skin permeation enhancers in the conducting medium. Permeation enhancers are used in topical and transdermal preparations with a view to disrupting the barrier properties of the skin so as to enhance the delivery of active agents through the skin. Some of these act to cause localised swelling and disruption of the stratum corneum, upper layer of the skin. These may be used in a synergistic manner with the conducting medium to reduce the resistance of movement of analytes from the skin to the patch which is used in conjunction with reverse iontophoresis. Skin permeation enhancers are very wide ranging and include essential oils, and alcohols as well as surfactants and vesicles and nanoparticulates, and are detailed throughout literature.

The ability to deposit liquid to achieve electrical contact will allow fluid to be directly delivered to the sensor downstream of the analyte collection chamber, thus reducing the costs and reliability issues associated with integrating a sensor in close proximity to the elements of the fluid withdrawal mechanism. The analytes may be drawn into a chamber 13 further into the patch, e.g. adjacent to the microelectronics layer 1, for analytics and data processing, allowing easier and cheaper product manufacture. The process of sampling for sensing and detection may be enhanced using this technique. Figure 2C indicates a screen or filter or membrane 14, i.e., a means of filtering the analyte by size or surface properties, between the electrolyte chamber and the conduit 11 leading to the sensing chamber 13. This will allow any rogue matter to be filtered out of the sample to be analysed, thus enhancing the purity of the analysed sample. The conduit 11 may be enlarged as indicated in figure 2d and additional electrodes 7a and 7b inserted, which may be stimulated to induce the movement of the analyte from the sampling chamber to the sensing chamber and increase its concentration in proximity with the sensor for detection. The conduit 11 may be prefilled with a conducting medium of appropriate viscosity and conductance to allow selective movement of analytes from the sampling chamber 9 to the sensing chamber 13, thus further improving the reliability and robustness of the system. A combination of the actuation mechanism, iontophoresis and fluids in the conduit may be used to flush the sensors between readings, or to transport analytes to different regions of the sensor at each reading interval.

Instead of a single conduit 11 leading to the sensing chamber 13 there may be a large number of micro-conduits designed to ensure iontophoretic movement of ions. As a result of osmotic effects, neutral molecules such as glucose may also be carried through the conduits 11, following the concentration gradient from the sampling chamber 9 to the sensing chamber 13.

The electrodes 7, 7a, 7b, may be formed of any of the commonly available electrode materials such as silver, silver-chloride, platinum, and electrodes produced from carbon nanotubes. Suitable sensors may be integrated for the detection of any number of analytes such as amino acids, sugars, fatty acids, co-enzymes, hormones, neurotransmitters, and drugs.

This versatility and enhanced sampling means will facilitate the detection of a number of potential analytes such as urea (for diagnosing chronic kidney disease) and blood lactate (for critical care patients) in addition to blood glucose monitoring.

The reservoir layer 3 may be composed of numerous large chambers 4 each measuring up to 10mm in diameter, or several hundred smaller chambers 4 each measuring a few micrometres in diameter. The actuator 2 may divided into sections, whereby under the control of the microelectronics layer 1 individual chambers 4 or groups of chambers 4 can be acted on selectively. In Fig. 1b, only one of the chambers 4 is being extended, as indicated by arrows 10. The material of composition of the reservoir chambers 4 may be polymeric, e.g. the Eudragit (Registered Trade Mark) range of pharmaceutical polymers sold by Röhm GmbH, acrylic acid cross-linked polymers, PDMS (polydimethylsiloxane), silicone, polyurethane and other polymeric materials that are deemed compatible.

As shown in Fig. 2, each chamber 4 may be in fluid communication with a single or multiple pores 8 passing through the resilient membrane 5. These pores may be created with or without material removal, the latter being to prevent leakage from the reservoirs on storage and upon administration prior to actuation of the reservoir, ranging from sub-micron to millimetre diameter. All transdermal systems generally have a backing layer applied during manufacture as a storage aid, and also to protect the patch, in particular where the patch may be a drug containing transdermal patch. The backing layer would essentially seal the face of the patch that would adhere to the skin, and various materials are commonly used including polypropylene, and paper/cardboard. We suggest here the use of a backing layer of a gelatinous composition with surface properties that will repel the solution in the patch thus providing a more robust seal. These materials will adhere firmly to the patch, and can be easily removed without leading to the presence of gel residues upon removal. Materials for such backing layers include silicone gel and hydrogels.

Instead of electrolyte solution, some of the reservoir chambers 4 may store drug formulations to be delivered direct to the surface of the skin according to a programmed regime under the control of the actuator 2, as described in patent application WO 2005/120471. The regime can be patient-centric or chrono-therapeutic, or based on a built-in non-invasive diagnostic monitoring mechanism with self regulating feedback/drug release, i.e. the analysis of the patient's interstitial fluids conducted in chambers 13 may be used to determine the quantity and timing of drugs delivered from the reservoirs. Feedback mechanisms, based on available technology, may be integrated and optimised to allow remote feedback of patient data to a central system/computer. In combination the above features provide a fully integrated robust system for diagnostics and therapy. Clearly, in this arrangement the actuator 2 must be capable of acting independently on the electrolyte-containing chambers 4 and the drug-containing chambers 4 but a common mechanism may be used for both. The drug may be carried by a conducting medium so that the electrodes 7 can be used, in the opposite sense from that previously described, to propel the drug molecules into the skin by forward iontophoresis.

As shown in Figures 2a and 2b, a micro-pump or other suction mechanism 12 may be placed in each of the conduits 11 to enhance withdrawal of analyte from the patient and delivery of it to the analysis chambers 13. Alternatively, a source of low pressure (not illustrated) - internal or external to the patch - may be connected to each of the analysis chambers 13 to assist with drawing fluid into them.

Similarly, a source of low pressure may be connected to the pores 8 in the resilient layer 5 to assist with drawing fluid (electrolyte or drug) out of the chambers 4. The invention encompasses embodiments in which control of relative pressures in this manner is the sole means for controlling transport of fluid from the reservoir chambers 4, i.e. embodiments in which the provision of an actuator that can extend or compress the reservoir layer is not required.

The patch need not be supplied as a single, indivisible unit. It may comprise two or more attachable and detachable segments in order to allow parts of the patch to be reused and other parts replaced. For example, the actuator may be integral with the reservoir layer but may be detachable from the microelectronic control and the power supply, whereby the power supply can be replaced when the batteries fail, without replacing the entire patch. Alternatively, the microelectronic control could be integral with the actuator so that only the power supply needs to be replaced. In a further alternative, the reservoir layer may be detachable from the actuator, which is integral with the control and power supply, whereby when the supply of drug in the reservoir is exhausted, it may be replaced (with suitable alignment of the reservoirs and conduits) without disposing of the still-functional microelectronics.

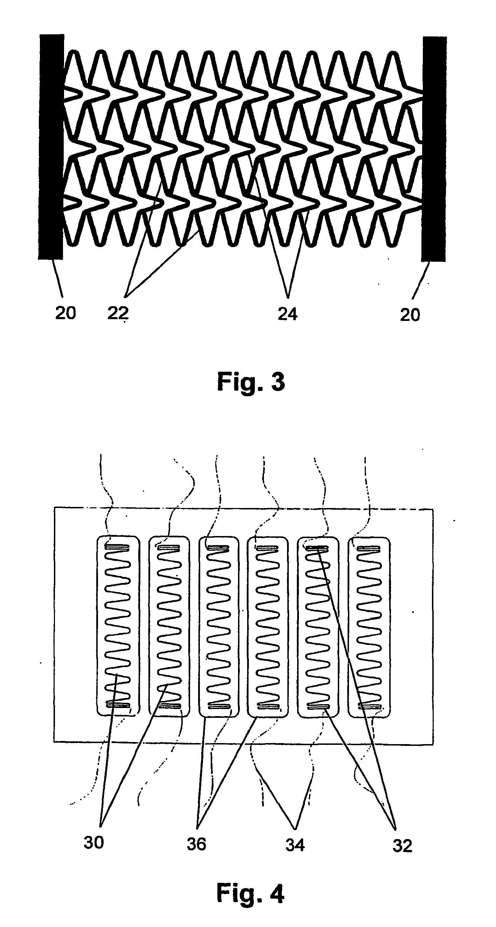

Figure 3 shows one example of an actuator that can be used to extend the reservoir layer 3 in patches according to the invention such as those shown in Figures 1 and 2. The illustrated actuator is formed in one piece from a sheet of shape memory alloy.

Shape memory alloys (SMAs) are materials that remember their geometry owing to a temperature-dependent phase transformation from a low-symmetry to a high-symmetry crystallographic structure, known as martensite and austenite structures respectively. There are three main types of SMAs: copper-zinc-aluminium, aluminium-nickel, and nickel-titanium. The latter possess superior mechanical properties. The biggest advantage of SMAs is their ability to produce both large forces and rapid displacements with low voltage requirements. The major downside is the poor energy efficiency and large hysteresis. However, for the purposes of this application the benefits of the large displacements and forces may outweigh the poor energy efficiency and inherent hysteresis since the system ideally calls for lateral displacement of tens to hundreds of microns, at a low rate.

SMAs are considered most suited to this particular application due to their physical and mechanical properties. Nickel-titanium due to its better mechanical properties is the preferred choice, and may be used in mesh form in an appropriate configuration to create the requisite lateral displacements.

Nickel-titanium (Nitinol) shape memory wire was obtained with the following characteristics:

| **Wire thickness (µm)** | **Transition temperature (°C)** | **Maximum pull force (g)** |
|---|---|---|
| 76.0 | 70 | 80 |
| 101.6 | 70 | 150 |
| 152.4 | 70 | 330 |

The wire was actuated to create approximately 5% strain in a 6cm x 3cm section of polymer film produced from Eudragit NE 30D acrylic polymer, approximately 0.7mm thick, using a bench top power supply to supply current through the wire. Voltage was recorded using a handheld digital voltmeter. The temperature of the wire was also recorded, together with transition time, i.e. the time taken for the polymer to revert back to its original length upon removal of the bias voltage. The results are indicated below.

| **Wire thickness (µm)** | **Voltage (V)** | **Operating temperature (°C)** | **Transition time (sec)** |
|---|---|---|---|
| 76.0 | < 0.3 | 38 -45 °C | <3 |
| 101.6 | < 0.5 | 38 - 45 °C | <3 |
| 152.4 | < 1.2 | 38 - 45 °C | <3 |

Based on the above results Nitinol wire of thickness 101.6 µm was selected for further studies due to its moderate power requirement and adequate pull force. In theory any of these thicknesses would be suited to the purpose here. It will be seen that the operating temperature is comparable with human body temperature (37°C) so will not be uncomfortable if applied close to the skin of a patient.

A number of designs were drawn up based on SMA wires. By bending the SMA wire to create a serpentine shape the strain effect of the SMA could be greatly enhanced. Trials showed that sufficient strain and force could be attained using this approach. Calculations showed that by making the SMA into a lever array, the displacement could be magnified to values that were consistent with the requirements of the patch.

To maximise the strain produced from the SMA it was decided to utilise the serpentine shape previously identified. It was decided to form the SMA actuator as a generally planar mesh, not built up from individual wires but cut from a single sheet of SMA using industrial standard processes. The process of laser cutting or chemical etching is used to mass manufacture micro components for devices such as cameras and analogue watches. This process is capable of producing tens of thousands components with tight tolerances in the hundreds of microns to centimetre size range. This processes used for the project have the advantage that they are relatively straightforward to scale up for production runs of considerable numbers as would be required for commercialisation.

Two processing routes - chemical etching and laser cutting - were explored as routes to produce the required structure. Both routes proved successful, with laser machining producing a superior finish for the scale of structure developed. Laser machining is more costly than chemical etching. Chemical etching may be more applicable for situations requiring smaller structures produced using thinner SMA sheet.

Figure 3 is a drawing of an actuator formed by laser cutting from a sheet of Nitinol shape memory alloy. It has the general form of a rectangle of dimensions approximately 2cm x 3cm having a pair of uncut bars 20 at its ends, between which extends a mesh of interconnected wires with holes between them. The mesh can be understood as four zigzag or serpentine wires 22 extending along the length of the actuator between the bars 20. In this example, the crest-to-crest wavelength of the zigzags is approximately equal to the amplitude of the zigzags measured crest-to-trough. The four zigzag wires 22 are mutually aligned so that the crests of one wire lie adjacent to the troughs of the neighbouring wire. Extending between each pair of adjacent crests and troughs of the respective zigzag wires 22 are bridging wires 24. The bridging wires 24 are bent or curved. In this example, all the bends of the bridging wires have the same orientation and the angle of the bends in the bridging wires 24 is approximately equal to the angle of the bends in the zigzag wires 22.

In use, a voltage is applied across the actuator by connecting a power supply to the respective end bars 20. A current flows through the wires of the mesh, resistively heating them, which causes the SMA to exceed its transition temperature and change the geometry of the mesh. The form of the zigzag wires 22 magnifies the change so that the dominant result is an increase in the length of the actuator. The primary purpose of the bridging wires 24 is to maintain the alignment between the respective zigzag wires 22 and to prevent them from deforming out of the plane of the mesh.

Using this device, a low temperature (martensitic), strain of up to 66 % was achieved without failure. The SMA design was also able to produce a force of at least 4.25 N when actuating from the low to the high temperature phase.

An alternative method of heating the actuator would be by chemical means, for example an exothermic oxidation reaction.

It will be understood that the mesh could be formed to have many different configurations while achieving a similar result. It will also be understood that similar configurations of mesh can be created by joining or interlocking strands of SMA wires instead of laser cutting, etching or stamping from a sheet.

Figure 4 shows a prototype actuator formed from discrete wires 30 of a shape memory alloy.

Each of the six actuator wires 30 extends in a serpentine or zigzag shape between two end bars 32. In the example shown, each end bar 32 is attached to a flexible control wire 34, which allows connection to suitable control electronics (not shown). In a practical patch, the control wires 34 could instead be embodied as part of a printed circuit board. When a current is applied through one of the wires 30, the wire is heated to above the transition temperature and its geometry changes to increase the overall length of the wire (i.e. the straight-line distance between the end bars 32). Each of the zigzag actuator wires 30 is contained within a generally flat pouch so that its deformation is constrained to lie within the plane of the device.

It will be understood that with suitable control electronics each of the wires 30 may be independently heated to selectively deform one or more associated reservoirs in an adjacent layer of the device (not shown in Fig. 4).

Furthermore it will be also understood that where shape memory metals are used as the actuator, they may be insulated and may be anchored to a rigid frame to provide leverage, and to prevent the body of the patch from buckling under the strain.

## Claims

1. A patch for sampling one or more analytes through the skin of a patient comprising:
an electrode layer (7) for positioning adjacent to the skin of a patient;
means (1) for actuating the electrode layer (7) to induce the withdrawal of analyte through the skin of the patient by reverse iontophoresis;
a first reservoir (4) containing an electrically conducting medium;
means (2) for controlling transport of the conducting medium from the first reservoir (4) onto a surface of the electrode layer (7) adjacent to the skin to increase the conductivity between the electrode layer and the skin; and
means for transporting analytes withdrawn from the skin of the patient to a location (13) where they are to be analysed.

2. A patch according to claim 1, wherein the means (2) for controlling the transport of the conducting medium from the reservoir (4) comprises means for applying positive pressure to the reservoir (4).

3. A patch according to claim 2, wherein the means for applying positive pressure to the reservoir (4) comprises an actuator means (2), which actuates on receipt of a control stimulus to stretch and/or compress the reservoir (4).

4. A patch according to claim 1, wherein the means for controlling the transport of the conducting medium from the reservoir (4) comprises means for applying negative pressure to a channel (8) downstream from the reservoir (4).

5. A patch according to any preceding claim, further comprising a second reservoir (4') containing a drug for transdermal delivery to the patient, wherein the means for controllably delivering conducting medium from the first reservoir (4) is also suitable for delivering the drug from the second reservoir (4') to the skin of the patient.

6. A patch according to claim 5, further comprising means for analysing the analyte sampled from the patient and for controlling the delivery of drug to the patient based on the results of the analysis.

7. A patch according to any preceding claim, wherein the means for transporting analytes to a location where they are to be analysed comprises at least one conduit (11) for delivering analytes from the skin of the patient to a sensing chamber (13).

8. A patch according to claim 7, comprising one or more further electrodes (7a,7b) adjacent to the conduit (11), which may be actuated to induce the flow of analytes through the conduit (11).

9. A patch according to claim 8, wherein the conduit (11) is pre-filled with an electrically conducting medium or is made from an electrically conductive material.

10. A patch according to any of claims 7 to 9, further comprising means (14) for filtering the analytes as they pass through the conduit.

11. A patch according to any preceding claim that is formed in at least two parts, one of the parts being removable from the patch for replacement.

12. A patch according to claim 11 wherein the removable part includes the first reservoir (4).

13. A method of sampling one or more analytes through the skin of a patient comprising the steps of:
positioning a patch such that an electrode layer (7) of the patch is adjacent to the skin of the patient;
transporting an electrically conducting medium from a first reservoir (4) in the patch onto a surface of the electrode layer (7) adjacent to the skin to increase the conductivity between the electrode layer and the skin;
actuating the electrode layer (7) to induce the withdrawal of analyte through the skin of the patient by reverse iontophoresis; and
transporting the analytes withdrawn from the skin of the patient to a location (13) where they are to be analysed.

14. A method according to claim 13, wherein the step of transporting the electrically conducting medium from the first reservoir (4) includes operating an actuator means (2) to expel liquid from the reservoir.

15. A method according to claim 14, wherein the step of positioning the patch includes adhering the patch to the skin of the patient; and
wherein the step of operating the actuator means (2) is effective to stretch the area of the patient's skin to which the patch is adhered.

## Patentansprüche

1. Pflaster zum Sammeln eines oder mehrerer Analyten durch die Haut,
mit einer Elektrodenschicht (7) zur Positionierung an der Haut eines Patienten,
mit einer Einrichtung (1) zum Betätigen der Elektrodenschicht (7), um das Herausziehen des Analytes durch die Haut des Patienten durch Umkehriontophorese herbeizuführen,
mit einem ersten Reservoir (4), welches ein elektrisch leitendes Medium enthält,
mit einer Einrichtung (2) zum Regeln des Transports des leitenden Mediums von dem ersten Reservoir (4) auf eine Oberfläche der Elektrodenschicht (7) in der Nähe der Haut, um die Leitfähigkeit zwischen der Elektrodenschicht und der Haut zu steigern, und
mit einer Einrichtung zum Transportieren von aus der Haut des Patienten heraus gezogenen Analyten an einen Ort, an dem diese analysiert werden sollen.

2. Pflaster nach Anspruch 1, bei dem die Einrichtung (2) zum Steuern des Transports des leitenden Mediums von dem Reservoir (4) eine Einrichtung zum Aufbringen eines positiven Drucks auf das Reservoir (4) aufweist.

3. Pflaster nach Anspruch 2, bei dem die Einrichtung zum Aufbringen eines positiven Drucks auf das Reservoir (4) eine Betätigungseinrichtung (2) aufweist, welche bei Empfang eines Steuerimpulses aktiv wird, um das Reservoir (4) zu strecken und/oder zu komprimieren.

4. Pflaster nach Anspruch 1, bei dem die Einrichtung zum Steuern des Transports des leitenden Mediums von dem Reservoir (4) eine Einrichtung zum Aufbringen von negativem Druck auf einen strömungsmäßig nach dem Reservoir (4) angeordneten Kanal (8) aufweist.

5. Pflaster nach einem der vorhergehenden Ansprüche, mit einem zweiten Reservoir (4'), welches ein Medikament für eine transdermale Abgabe zu dem Patienten enthält, wobei die Einrichtung zum gesteuerten Abgeben eines leitenden Mediums von dem ersten Reservoir (4) auch geeignet ist für die Abgabe des Medikaments von dem zweiten Reservoir (4') auf die Haut des Patienten.

6. Pflaster nach Anspruch 5, mit einer Einrichtung zum Analysieren des von dem Patienten genommenen Analyts und zum Regeln der Abgabe eines Medikaments zu dem Patienten, basierend auf den Ergebnissen der Analyse.

7. Pflaster nach einem der vorhergehenden Ansprüche, bei dem die Einrichtung zum Transportieren von Analyten zu einem Ort, an dem sie analysiert werden sollen, wenigstens eine Leitung (11) zum Abgeben von Analyten von der Haut des Patienten zu einer Messkammer (13) aufweist.

8. Pflaster nach Anspruch 7, mit einer oder mehreren weiteren Elektroden (7a, 7b) in der Nähe der Leitung (11), welche aktiviert werden können, um die Strömung der Analyte durch die Leitung (11) hervorzurufen.

9. Pflaster nach Anspruch 8, bei dem die Leitung (11) mit einem elektrisch leitenden Medium vorgefüllt oder aus einem elektrisch leitenden Material hergestellt ist.

10. Pflaster nach einem der Ansprüche 7 bis 9, mit einer Einrichtung (14) zum Filtern der Analyte, wenn sie durch die Leitung strömen.

11. Pflaster nach einem der vorhergehenden Ansprüche, welches aus wenigstens zwei Teilen hergestellt ist, wobei eines der Teile von dem Pflaster für einen Ersatz entfernbar ist.

12. Pflaster nach Anspruch 11, bei dem das entfernbare Teil das erste Reservoir (4) aufweist.

13. Verfahren zum Entnehmen eines oder mehrerer Analyten durch die Haut eines Patienten mit folgenden Schritten:
Positionieren eines Pflasters derart, dass eine Elektrodenschicht (7) des Pflasters an der Haut des Patienten anliegt,
Transportieren eines elektrisch leitenden Mediums aus einem ersten Reservoir (4) in dem Pflaster auf eine Oberfläche der Elektrodenschicht (7) auf der Haut, um die Leitfähigkeit zwischen der Elektrodenschicht und der Haut zu erhöhen,
Betätigen der Elektrodenschicht (7), um das Entnehmen eines Analyts durch die Haut des Patienten durch Umkehriontophorese herbeizuführen, und
Transportieren der entnommenen Analyte von der Haut des Patienten an einen Ort (13), wo sie analysiert werden sollen.

14. Verfahren nach Anspruch 13, bei dem der Schritt Transportieren des elektrisch leitenden Mediums von dem ersten Reservoir (4) ein Betreiben einer Betätigungseinrichtung (2) zum Ausstoßen einer Flüssigkeit von dem Reservoir beinhaltet.

15. Verfahren nach Anspruch 14, bei dem der Schritt Positionieren des Pflasters ein Anheften des Pflasters auf der Haut des Patienten aufweist, und bei dem der Schritt Betreiben der Betätigungseinrichtung (2) bewirkt, den Bereich auf der Haut des Patienten, auf welchem das Pflaster aufgeklebt wird, zu strecken.

## Revendications

1. Timbre pour prélever à travers la peau d'un patient des échantillons d'une ou de plusieurs substances à analyser, comprenant :
une couche d'électrode (7) destinée à être positionnée de manière adjacente à la peau d'un patient ;
des moyens (1) pour actionner la couche d'électrode (7) afin d'induire le retrait de la substance à analyser à travers la peau du patient par iontophorèse inversée ;
un premier réservoir (4) contenant un milieu électriquement conducteur ;
des moyens (2) pour contrôler le transport du milieu conducteur à partir du premier réservoir (4) sur une surface de la couche d'électrode (7) adjacente à la peau afin d'augmenter la conductivité entre la couche d'électrode et la peau ; et
des moyens pour transporter des substances à analyser retirées de la peau du patient jusqu'à un emplacement (13) où elles doivent être analysées.

2. Timbre selon la revendication 1, dans lequel les moyens (2) pour contrôler le transport du milieu conducteur à partir du réservoir (4) comprennent des moyens pour appliquer une pression positive sur le réservoir (4).

3. Timbre selon la revendication 2, dans lequel les moyens pour appliquer une pression positive sur le réservoir (4) comprennent des moyens d'activation (2) qui fonctionnent, suite à la réception d'un stimulus de commande pour étirer et/ou comprimer le réservoir (4).

4. Timbre selon la revendication 1, dans lequel les moyens pour contrôler le transport du milieu conducteur à partir du réservoir (4) comprennent des moyens pour appliquer une pression négative sur un canal (8) en aval du réservoir (4).

5. Timbre selon l'une quelconque des revendications précédentes, comprenant en outre un deuxième réservoir (4') contenant un médicament pour la distribution transdermique au patient, dans lequel les moyens pour distribuer de manière contrôlable le milieu conducteur à partir du premier réservoir (4) sont également appropriés pour distribuer le médicament à partir du deuxième réservoir (4') sur la peau du patient.

6. Timbre selon la revendication 5, comprenant en outre des moyens pour analyser la substance à analyser prélevée sur le patient et pour contrôler la distribution du médicament au patient en fonction des résultats de l'analyse.

7. Timbre selon l'une quelconque des revendications précédentes, dans lequel les moyens pour transporter les substances à analyser jusqu'à un emplacement où elles doivent être analysées comprennent au moins un conduit (11) pour distribuer les substances à analyser de la peau du patient à une chambre de détection (13).

8. Timbre selon la revendication 7, comprenant une ou plusieurs électrodes supplémentaires (7a, 7b) adjacentes au conduit (11) qui peuvent être actionnées pour induire l'écoulement des substances à analyser à travers le conduit (11).

9. Timbre selon la revendication 8, dans lequel le conduit (11) est pré-rempli avec un milieu électriquement conducteur ou est fabriqué à partir d'un matériau électriquement conducteur.

10. Timbre selon l'une quelconque des revendications 7 à 9, comprenant en outre des moyens (14) pour filtrer les substances à analyser au fur et à mesure qu'elles passent dans le conduit.

11. Timbre selon l'une quelconque des revendications précédentes, qui est formé en au moins deux parties, l'une des parties étant amovible du timbre pour être remplacée.

12. Timbre selon la revendication 11, dans lequel la partie amovible comprend le premier réservoir (4).

13. Procéder pour prélever des échantillons d'une ou de plusieurs substances à analyser à travers la peau d'un patient, comprenant les étapes consistant à :
positionner un timbre de sorte qu'une couche d'électrode (7) du timbre est adjacente à la peau du patient ;
transporter un milieu électriquement conducteur à partir d'un premier réservoir (4) dans le timbre sur une surface de la couche d'électrode (7) adjacente à la peau pour augmenter la conductivité entre la couche d'électrode et la peau ;
actionner la couche d'électrode (7) pour induire le retrait de la substance à analyser à travers la peau du patient par iontophorèse inversée ; et
transporter les substances à analyser retirées de la peau du patient jusqu'à un emplacement (13) où elles doivent être analysées.

14. Procédé selon la revendication 13, dans lequel l'étape consistant à transporter le milieu électriquement conducteur à partir du premier réservoir (4) comprend l'étape consistant à actionner des moyens d'activation (2) pour expulser le liquide du réservoir.

15. Procédé selon la revendication 14, dans lequel l'étape consistant à positionner le timbre comprend l'étape consistant à coller le timbre sur la peau du patient ; et
dans lequel l'étape consistant à actionner les moyens d'activation (2) est efficace pour étirer la surface de la peau du patient sur laquelle le timbre est collé.
